# EUROPEAN PATENT APPLICATION

(11) **EP 1 882 939 A1**
(43) Date of publication of application: **30.01.2008**
(21) Application number: 06015676.7
(22) Date of filing: 27.07.2006
(51) Int. Cl.: G01N 33/543

(54) **Urinary immunochromatographic antigen detection cup**

(71) Applicant: The Jordanian Pharmaceutical Manufacturing Co., 11710 Naor (JO)
(72) Inventor: Adnan, Badwan, Dr., 11185 Amman (JO); Murshed, Abedel-qader Mohammed, 11118 Amman (JO)
(74) Representative: Winkler, Andreas Fritz Ernst

(57) **Abstract**

The present invention refers to an immunochromatographic detection cup and its use for detecting antigens in urine indicative of tuberculosis, *Leishmania* infection, malaria, *Schistosoma* infection and pneumonia.

## Description

The present invention refers to an immunochromatographic detection cup and its use for detecting antigens in urine indicative of tuberculosis, *Leishmania* infection, malaria, *Schistosoma* infection and pneumonia.

### Background of the Invention

Tuberculosis (TB) is a major and increasing public health problem in both industrialized and developing countries. Hence, the development of new inexpensive, rapid and field adapted methods for its diagnosis is urgently needed. Sputum culture, which is still the reference method for the diagnosis of pulmonary TB, is cumbersome and time-consuming, and requires access to expensive biosafety level 3 (BSL3) laboratories. Microscopy of direct smears for acid-fast bacilli (AFB) as recommended by WHO for developing countries is the most commonly used method for diagnosis of TB. A major disadvantage with this method is its low sensitivity, even after concentration of the sputum samples.

The availability of new field adapted, low-cost, and rapid diagnostic tests to supplement AFB microscopy, and especially methods improving the diagnosis in AFB-negative disease, would be of great benefit for TB control programs, in particular in areas lacking appropriate safety laboratories. Among the newly developed methods for rapid diagnosis of TB, nucleic acid amplification methods such as PCR seem most promising, but the technology is still too complex to be feasible for TB control programs in developing countries. Antibodies against a number of mycobacterial antigens have been identified in patients using a variety of immunological techniques, but no antibody test has so far reached sufficient sensitivity and/or specificity for routine diagnostic purposes. Detection of circulating or secreted *Mycobacterium tuberculosis* antigens seems attractive and has been explored in a number of studies. However, no satisfactory commercial test for mycobacterial antigens in serum or sputum is currently available.

The idea of identifying mycobacterial antigens in urine of TB patients is attractive for several reasons: urine is more readily obtainable than serum samples and urinary specimens do not carry the risks inherent to needles and blood-based laboratory work. Furthermore, if the urine specimens are boiled before handling, there is no need for BSL3 facilities.

In 1920s, mycobacterial antigens were detected in the urine of TB patients, and the diagnostic potential of such antigens was subsequently discussed by other scientists. More recently, the diagnostic value of mycobacterial antigens in the urine of leprosy patients has been assessed. Unfortunately, the techniques involved turned out to be insufficiently sensitive in paucibacillary patients, the patient group where improved diagnostic tests are most needed.

Lipoarabinomannan (LAM) is a major and structurally important glycolipid component of the outer cell wall of all mycobacteria and may account for up to 15% of the total bacterial weight. LAM is a carbohydrate antigen with glycosidic linkages for which no human degrading glycosidases are known. Hence, we assumed that in active mycobacterial disease LAM may be cleared through the kidneys and occur in urine in antigenically intact form. Furthermore, since LAM is a carbohydrate antigen and thus inherently heat-stable, LAM may be detectable by sensitive immunological techniques, even after boiling of the urine. At least theoretically, the amount of LAM in the urine should reflect the bacterial load, metabolic activity and/or rate of degradation of the bacteria, and hence permit a semi-quantitative assessment of the infectious status. A high sensitive, simple, fast and method for LAM detection and quantification was reported using an enzyme-linked immunosorbent assay (ELISA) in AFB positive sputa from TB patients ¹.

However, it would be of particular advantage to have rapid immunochromatographic test devices available for detecting LAM in urine.

In recent years the *in vitro* diagnostics (IVD) industry has made enormous efforts to develop immunochromatographic tests. Such tests have found applications in both clinical and non-clinical fields ². A clinical utility of this test format has been shown for more than 150 different analytes, and many of them are target now of commercially available diagnostic products ³. The wide range of applications for such devices has been reviewed ^{2,4}.

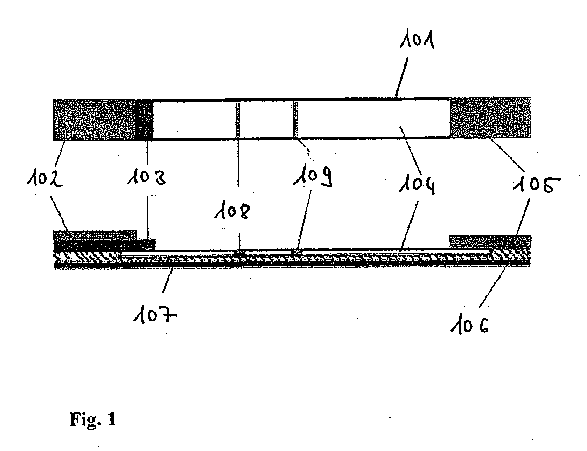

Rapid immunochromatographic test devices, e.g. in the form of a test strip, are made up of a number of components (see Fig. 1). Such a test strip 101 commonly includes a sample pad 102, a conjugate pad 103, a membrane 104, e.g. a nitrocellulose membrane, and an absorbent pad 105. The membrane 104 is usually attached by means of an adhesive 106 to a supporting backing 107, e.g. made of plastic. In practice, the user dispense a patient sample (usually urine or whole blood) onto the sample pad 102.The sample then flows through the sample pad 102 into the conjugate pad 103, where it mixes with and releases the detector reagent. This mixture then flows across the membrane 104, where it binds with the test and control reagents located in the capture test zone 108 and negative control zone 109, respectively. When the mixture binds to the reagent that forms the test line, a positive result is indicated. The colour intensity of the test line is proportional to the concentration of analyte in the sample. Excess sample that flows beyond the test and control zones 108, 109 is taken up in the absorbent pad 105.

Rapid immunochromatographic test devices for diagnostic purposes are easy to operate and thus do not only contribute to the comfort of professional users, e.g. medical stuff, but also allow the operation by non-professionals users, e.g. most patients.

The object of the present invention is to provide a rapid immunochromatographic test device for detecting LAM and other antigens indicative of bacterial or parasitic infections in urine specimens.

### Summary of the Present Invention

The object of the present invention is solved by a urinary immunochromatographic detection cup comprising:
(a) a sample-collecting container;
(b) at least one test device inside the container, which test device comprises at least one capture antibody directed against an antigen to be detected and/or at least one non-specific control antibody;
(c) at least one conjugate releasing pad separated from the test device, which conjugate releasing pad comprises at least one antibody colloidal gold conjugate;
(d) at least two sample absorbent pads linked to the test device at different positions.

In one embodiment, the test device further comprises a nitrocellulose membrane on which the capture antibody and/or the control antibody is immobilized.

In one embodiment, the antigen to be detected is selected from a *Mycobaterium* antigen, *Leishmania* antigen, *Plasmodium* antigen, *Schistosoma* antigen and *Pneumonia* antigen.

In a preferred embodiment, the *Mycobacterium* antigen is lipoarabinomannan (LAM), the *Leishmania* antigen is urinary *Leishmania donovani* antigen, the *Plasmodium* antigen is *Plasmodium falciparum* histidine rich protein 2 (HRP-II), the *Schistosoma* antigen is *Schistosoma mansoni* antigen, and the *Pneumonia* antigen is pneumolysin (PLY)

In one embodiment, the control antibody is anti-mouse IgG.

In one embodiment, the antibody colloidal gold conjugate comprises a specific antibody directed against the specific antigen to be detected which antibody is specific for different antigen recognition site than that of the capture antibody, i.e. for example a pair of specific monoclonal antibodies for a specific antigen.

In one embodiment, the conjugate releasing pad is located at the internal surface of the container wall.

In one embodiment, a first sample absorbent pad is located at the bottom and a second sample pad is located at the top of the container.

In one embodiment, the sample absorbent pad at the top is fitted into a container cap.

In one embodiment, the sample absorbent pad at the top has a capacity of absorbing at least 15 ml of sample.

The object of the present invention is further solved by a use of the urinary immunochromatographic detection cup according to the present invention for detecting an antigen selected from a *Mycobaterium* antigen, *Leishmania* antigen, *Plasmodium* antigen, *Schistosoma* antigen and *Pneumonia* antigen in urine of a subject, preferably human.

In one embodiment of the use, the *Mycobacterium* antigen is lipoarabinomannan (LAM), the *Leishmania* antigen is urinary *Leishmania donovani* antigen, the *Plasmodium* antigen is *Plasmodium falciparum* histidine rich protein 2 (HRP-II), the *Schistosoma* antigen is *Schistosoma mansoni* antigen, and the *Pneumonia* antigen is pneumolysin (PLY).

In one embodiment of the use, the volume of the urine sample is approximately 15 ml.

In one embodiment, the detection cup is used for the diagnosis and monitoring of a subject affected by tuberculosis, *Leishmania* infection, malaria, *Schistosoma* infection and pneumonia.

Thus, by using a large volume of urine as a sample (15 ml), the present invention provides a rapid immunochromatographic test device for detecting bacterial or parasitic antigens in urine indicative of tuberculosis, *Leishmania* infection, malaria, *Schistosoma* infection and pneumonia. In particular, the urinary immunochromatographic detection cup provides a sensitive technique for the detection of LAM in early tuberculosis infection (down to concentration of 2 pg/ml).

### Detailed Description of the Invention

### Brief Description of the Figures

- Figure 1: shows top and side views of a typical rapid-flow immunochromatographic test device in the form of a test strip 101 including a sample pad 102, a conjugate pad 103, a membrane 104, an absorbent pad 105, an adhesive 106, a supporting backing 107, a test zone 108, and a control zone 109.
- Figure 2a: shows an assembly comprising a test strip 201, an absorbent sample pad 202, and an absorbent pad 205.
- Figure 2b: shows an immunochromatographic detection cup comprising a sample-collecting container 210, a cap 211 and the assembly shown in Fig. 2a.

### EXAMPLES

### EXAMPLE 1: Urinary immunochromatographic antigen detection cup

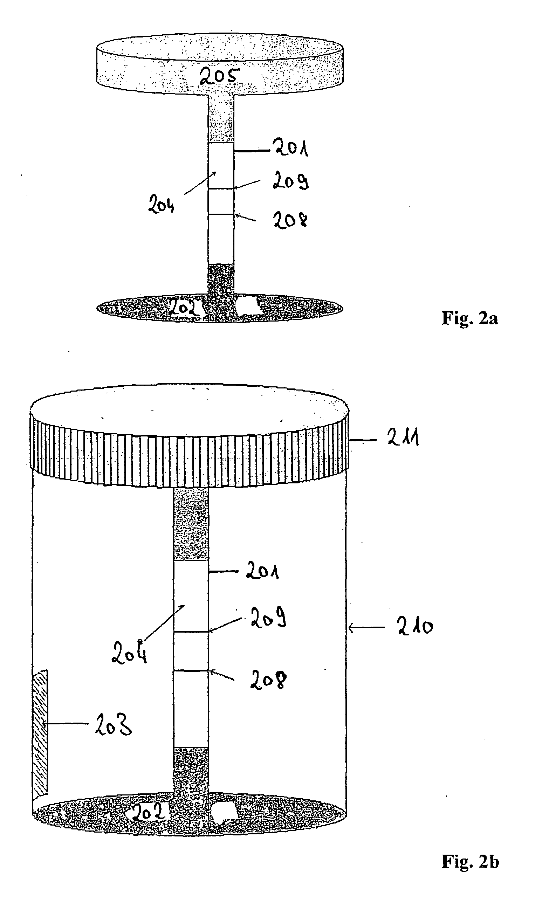

One embodiment of the rapid immunochromatographic detection system of the present invention is shown in Fig. 2a, b. The immunochromatographic detection cup comprises a sample-collecting container 210, actually the "cup", a cap 211 for closing the container, and a detection test strip 201 inserted inside the transparent container wall 210. The test result can be read on the outer surface. The test strip 201 comprises a nitrocellulose membrane 204 and is linked via an absorbent pad 202 on the test strip 201 to an absorbent sample pad 202 placed on the bottom of the container 210. The absorbent sample pad 202 is designed to cover the inner surface of the container bottom. At the opposite end, the test strip 201 is linked via an absorbent pad 205 on the test strip to an absorbent pad 205 which is fitted into the cap 211 of the container and is designed to be thick enough to absorb more than 15 ml of sample. A monoclonal antibody gold conjugate releasing pad 203 is fixed to the internal surface of the container wall. The monoclonal antibody gold conjugate, e.g. monoclonal anti-LAM (clone 1) gold conjugate (*cf.* Example 2 below), will begin releasing during the urine sample streaming into the container 210. Non-specific antibody is immobilized as a control zone 209 onto the nitrocellulose membrane 204 for non-specific capturing of the monoclonal antibody gold conjugate, while monoclonal antibody, e.g. monoclonal anti-LAM (clone 2) (cf. Example 2 below), are immobilized as a sample zone 208 for specific capturing of the antigen to be detected. In the embodiment shown in Fig. 2a, b the sample zone 208 and the control zone 209 are realized by a sample and control line, respectively. More than one sample zone 208 and/or more than one control zone 209 are also contemplated.

### EXAMPLE 2: Urinary tuberculosis antigen detection cup

Mouse monoclonal anti-LAM (clone 1) colloidal gold conjugate is comprised by the conjugate releasing pad 203 immobilized onto the internal surface of the sample-collecting container 210. The sample zone 208 comprises mouse monoclonal anti-LAM (clone 2) immobilized onto the nitrocellulose membrane 204. The control zone 209 comprises anti-mouse IgG. Sample zone 208 and control zone 209 turn into purple colour in case of tuberculosis LAM is present in the sample; only the control zone 209 turns into purple colour in case of tuberculosis LAM free sample.

### EXAMPLE 3: Urinary Leishmania antigen detection cup

Mouse monoclonal anti-urinary *Leishmania donovani* antigen (clone 1) colloidal gold conjugate is comprised by the conjugate releasing pad 203 immobilized onto the internal surface of the sample-collecting container 210. The sample zone 208 comprises mouse monoclonal anti-urinary *Leishmania donovani* antigen (clone 2) immobilized onto the nitrocellulose membrane 204. The control zone 209 comprises anti-mouse IgG. Sample zone 208 and control zone 209 turn into purple colour in case that urinary *Leishmania donovani* antigen is present in the sample; only the control zone 209 turns into purple colour in case of urinary *Leishmania donovani* antigen free sample.

### EXAMPLE 4: Urinary malaria antigen detection cup

Mouse monoclonal anti-*Plasmodium falciparum* histidine rich protein 2 (HRP-II) (clone 1) colloidal gold conjugate is comprised by the conjugate releasing pad 203 immobilized onto the internal surface of the sample-collecting container 210. The sample zone 208 comprises mouse monoclonal anti-*Plasmodium falciparum* histidine rich protein 2 (HRP-II) (clone 2) immobilized onto the nitrocellulose membrane 204. The control zone 209 comprises anti-mouse IgG. Sample zone 209 and control zone 208 turn into purple colour in case that *Plasmodium falciparum* histidine rich protein 2 (HRP-II) is present in the sample; only the control zone 209 turns into purple colour in case of *Plasmodium falciparum* histidine rich protein 2 (HRP-II) free sample.

### EXAMPLE 5: Urinary Schistosoma mansoni antigen detection cup

Mouse monoclonal anti-*Schistosoma mansoni* (clone 1) colloidal gold conjugate is comprised by the conjugated releasing pad 203 immobilized onto the internal surface of the sample-collecting container 210. The sample zone 208 comprises mouse monoclonal anti-*Schistosoma mansoni* (clone 2) immobilized onto the nitrocellulose membrane 204. The control zone 208 comprises anti-mouse IgG. The sample zone 209 and control zone 208 turn into purple colour in case that *Schistosoma mansoni* antigen are present in the sample; only the control line 209 turns into purple colour in case of *Schistosoma mansoni* antigen free sample.

### EXAMPLE 6: Urinary pneumonia antigen detection cup

Mouse monoclonal anti-pneumolysin (PLY) (clone 1) colloidal gold conjugate is comprised by the conjugate releasing pad 203 immobilized onto the internal surface of the sample-collecting container 210. The sample zone 208 comprises mouse monoclonal anti-pneumolysin (PLY) (clone 2) immobilized onto the nitrocellulose membrane 204. The control zone 208 comprises anti-mouse IgG. The sample zone 208 and control zone 209 turn into purple colour in case that pneumolysin (PLY) is present in the sample; only the control zone 209 turns into purple colour in case of pneumolysin (PLY) free sample.

### References

(1) Journal of Microbiological Methods, 45, 2001: 41-52
(2) J Chandler, N Robinson, and K Whiting, "Handling False Signals in Gold-Based Rapid Tests", IVD Technology 7, no. 2 (2001): 34-45; http://www.devicelink.com/ivdt/archive/0 1 /03/002.html.
(3) TC Tisone et al., "Image Analysis for Rapid-Flow Diagnostics", IVD Technology 5, no. 5(1999):52-58; http://www.devicelink.com/ivdt/archive/99/09/010.html.
(4) J Chandler, T Gurmin, and N Robinson, "The Place of Gold in Rapid Tests", IVD Technology 6, no. 2 (2000): 37-49; http://www.devicelink.com/ivdt/archive/00/03/004.html

## Claims

1. An urinary immunochromatographic detection cup comprising:
(a) a sample-collecting container;
(b) at least one test device inside the container, which test device comprises at least one capture antibody directed against an antigen to be detected and/or at least one non-specific control antibody;
(c) at least one conjugate releasing pad separated from the test device, which conjugate releasing pad comprises at least one antibody colloidal gold conjugate;
(d) at least two sample absorbent pads linked to the test device at different positions.

2. The detection cup according to claim 1, wherein the test device further comprises a nitrocellulose membrane on which the capture antibody and/or the control antibody is immobilized.

3. The detection cup according to claim 1 or 2, wherein the antigen to be detected is selected from a *Mycobaterium* antigen, *Leishmania* antigen, *Plasmodium* antigen, *Schistosoma* antigen and *Pneumonia* antigen.

4. The detection cup according to any of the preceding claims, wherein the *Mycobacterium* antigen is lipoarabinomannan (LAM), the *Leishmania* antigen is urinary *Leishmania donovani* antigen, the *Plasmodium* antigen is *Plasmodium falciparum* histidine rich protein II (HRP-II), the *Schistosoma* antigen is *Schistosoma mansoni* antigen, and the *Pneumonia* antigen is pneumolysin (PLY)

5. The detection cup according to any of the preceding claims, wherein the control antibody is anti-mouse IgG.

6. The detection cup according to any of the preceding claims, wherein the antibody colloidal gold conjugate comprises a specific antibody directed against the specific antigen to be detected which antibody is specific for different antigen recognition site than that of the capture antibody.

7. The detection cup according to any of the preceding claims, wherein the conjugate releasing pad is located at the internal surface of the container wall.

8. The detection cup according to any of the preceding claims, wherein a first sample absorbent pad is located at the bottom and a second sample pad is located at the top of the container.

9. The detection cup according to claim 8, wherein the sample absorbent pad at the top is fitted into a container cap.

10. The detection cup according to claim 8 or 9, wherein the sample absorbent pad at the top has a capacity of absorbing at least 15 ml of sample.

11. A use of the urinary immunochromatographic detection cup according to any of claims 1 to 10 for detecting an antigen selected from a *Mycobaterium* antigen, *Leishmania* antigen, *Plasmodium* antigen, *Schistosoma* antigen and *Pneumonia* antigen in urine of a subject, preferably human.

12. The use according to claim 11, werein the *Mycobacterium* antigen is lipoarabinomannan (LAM), the *Leishmania* antigen is urinary *Leishmania donovani* antigen, the *Plasmodium* antigen is *Plasmodium falciparum* histidine rich protein II (HRP-II), the *Schistosoma* antigen is *Schistosoma mansoni* antigen, and the *Pneumonia* antigen is pneumolysin (PLY).

13. The use according to claim 11 or 12, wherein the volume of the urine sample is approximately 15 ml.

14. The use according to any of claims 11 to 13 for the diagnosis and monitoring of a subject affected by tuberculosis, *Leishmania* infection, malaria, *Schistosoma* infection and pneumonia.
